Europäisches Patentamt

European Patent Office

Office européen des brevets

(19)

(11) Publication number: **0 019 454**
**B1**

(12) **EUROPEAN PATENT SPECIFICATION**

(45) Date of publication of patent specification: **16.02.83**

(51) Int. Cl.³: **C 07 C 87/58, C 07 C 85/11**

(21) Application number: **80301573.4**

(22) Date of filing: **14.05.80**

(54) **Manufacture of toluene diamine.**

(30) Priority: **14.05.79 US 38976**

(43) Date of publication of application:
**26.11.80 Bulletin 80/24**

(45) Publication of the grant of the patent:
**16.02.83 Bulletin 83/7**

(84) Designated Contracting States:
**DE FR GB**

(56) References cited:
**DE - A - 1 915 272**
**US - A - 2 976 320**

(73) Proprietor: **AIR PRODUCTS AND CHEMICALS, INC.**
**P.O. Box 538**
**Allentown, Pennsylvania 18105 (US)**

(72) Inventor: **Kunz, Nance Dicciani**
**4 Hilltop Road, RD 7**
**Bethlehem, Pennsylvania, 18015 (US)**
Inventor: **Johnson, Thomas Albert**
**RD 1**
**Orefield, Pennsylvania, 18069 (US)**
Inventor: **Milligan, Barton**
**RD 1**
**Coplay, Pennsylvania, 18037 (US)**

(74) Representative: **Lucas, Brian Ronald et al,**
**c/o Air Products Limited Hersham Place Molesey Road**
**Walton-on-Thames Surrey, KT12 4RZ (GB)**

Courier Press, Leamington Spa, England.

# Manufacture of toluene diamine

This invention relates to a process for producing toluene diamine from crude dinitrotoluene.

Crude dinitrotoluene is commonly prepared by the nitration of toluene by nitric acid in the presence of sulphuric acid. The resulting crude dinitrotoluene contains a small amount of acid together with *inter alia* nitrophenolic material, typically cressols, as byproduct.

Commercially toluene diamine is produced by reacting, in the presence of a catalyst, hydrogen and crude dinitrotoluene from which the nitrophenolic material has been removed. This removal has been effected by washing the crude dinitrotoluene with a dilute aqueous alkali solution, for example sodium carbonate solution and has been considered extremely important to inhibit poisoning of the catalyst and to inhibit decomposition of the toluene diamine formed. The importance previously attached to the removal of the nitrophenolic material is reflected in cited US—A—2,976,320. In particular, in this specification, which describes the catalytic hydrogenation of dinitrobenzene the applicants state that "We have discovered that nitrophenols and nitrocresols normally present in commercial dinitrotoluenes are powerful catalyst poisons and decomposition accelerators and that their concentration in such dinitro products is critically related to the safety and efficiency with which the dinitrotoluenes can be hydrogenated to the corresponding diamines. We have found that—by limiting the content of nitrophenols and nitrocresols to not more than 500 ppm—the latter can be hydrogenated safely and efficiently under ordinary or slightly elevated pressures in the molten state at temperatures as high as 150°C—in the presence of a hydrogenation catalyst such as nickel, platinum and palladium". The applicants later recommend washing the crude dinitrotoluene with dilute aqueous alkali to remove the nitrocresols and nitrophenols to the desired level.

We have discovered that the removal of this nitrophenolic material is in fact largely unnecessary and that the essential feature to inhibit catalyst poisoning and decomposition of the toluene diamine formed is to reduce the concentration of acid in the crude dinitrotoluene to less than 6000 ppm (by weight) (based on $HNO_3$) and preferably as low as possible. This can be achieved by simply washing the crude toluene diamine with water. The use of dilute aqueous alkali solutions to remove the nitrophenolic material is then redundant.

According to the present invention there is provided a method of manufacturing toluene diamine from crude dinitrotoluene containing acid together with *inter alia* nitrophenolic material characterized in that it comprises the steps of washing said crude dinitrotoluene with water until the concentration of acid in the crude dinitrotoluene is less than 6000 ppm (by weight) (based on $HNO_3$), and reacting the washed crude dinitrotoluene with hydrogen in a reactor in the presence of a catalyst without any intermediate step to reduce the concentration of nitrophenolic material present.

The crude dinitrotoluene is preferably washed with water to remove as much acid as is reasonably possible and we strongly recommend that the concentration of acid in the crude dinitrotoluene be reduced to not more than 2500 ppm (by weight) (based on $HNO_3$) and preferably much less.

The present invention is applicable to both the batch and continuous manufacture of toluene diamine. However, continuous manufacture of toluene diamine is particularly important. In such a case the concentration of dinitrotoluene in the contents of the reactor is preferably maintained between 0.0001 and 25% (by weight) and more preferably between 0.01 and 3% (by weight). Similarly, the concentration of catalyst in the contents of the reactor is preferably maintained between 0.1 and 25% (by weight) and more preferably between 0.1 and 3% (by weight).

The catalyst can comprise any of the various non-precious metal catalyst which are known for this purpose, for example nickel, nickel promoted by cobalt, chromium, molybdenum and iron with Raney nickel being preferred.

The catalyst may be supported on, for example kieselguhr, silica, silica-alumina or alumina although the Raney nickel is preferably unsupported.

The following examples are provided to illustrate the invention and all the parts and percentages are by weight unless otherwise specified.

Example 1

A 1 litre Parr 316 stainless steel reactor was charged with 500 ml of a mixture consisting of 60% toluene diamine, 38% water and 2% Raney nickel catalyst containing particles from 1 to 350 microns in diameter. The contents were then heated to 130°C and pressurized with hydrogen to 11.25 kg/cm².

An average of 1.454 gms/minute of crude dinitrotoluene which had been treated with sodium carbonate solution in accordance with the prior art and with an analysis which was probably similar to that set out in Example 4 was then fed to the reactor in a series of rapid pulses over a period in excess of 10 hours. Product was withdrawn from the reactor at the same rate as the crude dinitrotoluene was introduced so that the volume of the contents of the reactor remained constant.

The maximum concentration of crude dinitrotoluene in the reactor was approximately 0.3% and the average residence time in the reactor was 1.25 minutes.

Various measurements were made and are set out in Table 1.

2

# 0 019 454

Example 2

The procedure of Example 1 was repeated except that water washed crude dinitrotoluene having an analysis of:

| Dinitrotoluene | 99.0% |
| Nitrophenolic material | 0.080% |
| Nitric acid | 1200 ppm |
| Balance | approximately 1% |

was used in place of the crude dinitrotoluene which had been treated with sodium carbonate.

Various measurements were made and are set out in Example 2. In this connection it will be noted that no attempt was made to reduce the level of nitrophenolic material.

## TABLE 1

| | Example #1 control | Example #2 WW DNT |
|---|---|---|
| Temperature (°C) | 130 | 130 |
| Pressure (Kg/cm$^2$) | 11.25 | 11.25 |
| Catalyst Conc. (wt. %) | .4 | .4 |
| Vapor phase H$_2$ concentration (%) | 100 | 100 |
| Catalyst feed mode | batch | batch |
| Initial activity $\dfrac{\text{gmol H}_2}{\text{(min) (gm cat) (cm}^3\text{)}}$ | $1.37 \times 10^{-4}$ | $9.34 \times 10^{-5}$ |
| Tar generation (%) | 0.2—1.3% | 0.1—2% |
| Turnover $\dfrac{\text{part DNT}}{\text{hr. part cat.}}$ | 43.62 | 43.62 |
| Catalyst life $\dfrac{\text{part DNT}}{\text{part cat}}$ | 1975 | 1885 (minimum)[a] |
| Deactivation rate | $\dfrac{-.0086\% \text{ initial rate}}{\text{gm DNT reduced}}$ | $\dfrac{-.0091\% \text{ initial rate}}{\text{gm DNT reduced}}$ |
| Material balance closure (%) | 94.7 | 97.1 |

[a] Experiment terminated at this point; exhausted dinitrotoluene feed stock.

The results of Example 2 shows that water washed dinitrotoluene can be hydrogenated with negligible adverse effect on catalyst life or rate of reaction. The catalyst performance in Example 2 was lower probably only because of the exhausted feedstock. Tar generation was slightly higher in Example 2. This value may not represent any greater loss of material since tar generation in Example 1 does not include the nitrophenolic material removed by alkali treatment.

Examples 3 and 4

The procedure of Examples 1 and 2 was repeated except that different feed and higher catalyst concentrations were used.

The analysis of the alkaline treated dinitrotoluene feed (control) and the water washed dinitrotoluene feed was as follows:

### Feed compositions

| Analysis/Sample | Example #3 control DNT feed | Example #4 WW DNT feed |
|---|---|---|
| Water (ppm) | 4400 | 4800±100 |
| Freezing pt. (°C) | 56.2±.1 | 57.1±0.1 |
| Acidity (ppm as HNO$_3$) | 0.65 | 1928±128 |
| Alkalinity (ppm as KOH) | Nil | Nil |
| Cresols (ppm) | 8±1 | 700±10 |
| 2,4,6 TNT (ppm) | 730±40 | 1100±30 |
| nitrobenzene (wt. %) | 0.01 | 0.02 |

3

Feed compositions (cont.)

| Analysis/Sample | Example #3 control DNT feed | Example #4 WW DNT feed |
|---|---|---|
| o-nitrotoluene (wt. %) | 0.02 | 0.01 |
| m-nitrotoluene (wt. %) | 0.01 | 0.01 |
| p-nitrotoluene (wt. %) | 0.02 | 0.01 |
| 2,6 Dinitrotoluene (wt. %) | 19.14 | 17.73 |
| 2,5 Dinitrotoluene (wt. %) | 0.75 | 0.68 |
| 2,4 Dinitrotoluene (wt. %) | 75.44 | 77.17 |
| 3,5 Dinitrotoluene (wt. %) | 0.03 | 0.03 |
| 2,3 Dinitrotoluene (wt. %) | 1.62 | 1.47 |
| 3,4 Dinitrotoluene (wt. %) | 2.46 | 2.23 |
| Normalized isomer ratio 2,4/2,6 | 79.7/20.2 | 81.3/18.7 |
| Nitrate (ppm) | 0.3 | 1092±20 |
| Nitrite (ppm) | 0.35±.07 | 160±8 |
| Sulfate (ppm) | 0.69±0.26 | 230±50 |
| Oxalate (ppm) | 0.2 | 170±44 |
| Acetate (ppm) | 2.47±0.4 | |

The results of Examples 3 and 4 are given in Table 2.

TABLE 2

| | Example #3 control | Example #4 WW DNT |
|---|---|---|
| Temperature (°C) | 130 | 130 |
| Pressure (Kg/cm²) | 11.25 | 11.25 |
| Catalyst conc. (wt. %) | 1.7 | 1.7 |
| Vapor phase $H_2$ concentration (%) | 100 | 100 |
| Catalyst feed mode | batch | batch |
| Initial activity $\dfrac{gmol\ H_2}{(min)\ (gm\ cat)\ (cm^3)}$ | $3.67 \times 10^{-5}$ | $3.67 \times 10^{-5}$ |
| Tar generation (%) | .41—1.28% | .48—1.75% |
| Turnover $\dfrac{part\ DNT}{hr.\ part\ cat.}$ | 21.03 | 21.03 |
| Material balance closure (%) | 95.5 | 96.8 |

The results in terms of catalyst activity and reaction rate for both the control and the water washed dinitrotoluene appeared to be similar. Slightly higher tar generation with the water washed dinitrotoluene was noted. It was also noted the reaction was relatively insensitive to nitrophenolic material even though the concentration was about 700 ppm. Table 3 below is an analysis of the percentage tar versus the grams of dinitrotoluene (DNT) reduced per gram of catalyst.

TABLE 3

Tar analyses (wt. %)

| Gms. DNT reduced per gm. catalyst | % reaction mass produced from feed | Example #3 control | | Example #4 WW DNT | |
|---|---|---|---|---|---|
| | | % tar | DNT (ppm) | % tar | DNT (ppm) |
| 0 | 0 | 0.412 | 5.0 | 0.482 | 5.0 |
| 59.5 | 51.9 | 1.02 | 5.0 | 0.888 | 5.0 |
| 116.8 | 76.4 | 1.28 | 5.0 | 0.99 | 5.0 |
| 175.3 | 88.6 | 1.108 | 5.0 | 1.30 | 5.0 |
| 233.2 | 94.4 | 1.27 | 5.0 | 1.54 | 5.0 |
| 289.1 | 97.24 | 0.094 | 5.0 | 1.66 | 5.0 |
| 300.2 | 97.7 | 1.28 | 5.0 | 1.75 | 5.0 |

Table 3 shows that the tar concentration is slightly higher for the water washed dinitrotoluene feedstock particularly at higher levels of dinitrotoluene reduced per gram of catalyst. Even so the percent tar produced is relatively insignificant in terms of the benefit gained, i.e. the ability to eliminate the alkaline treatment of the crude dinitrotoluene and associated high chemical cost. Also, the process reduces the environmental problems associated with the alkaline treatment.

Examples 5 and 6

The procedure of Examples 1 and 2 was repeated in order to determine the sensitivity of the process to acid concentration. In Example 5 water washed dinitrotoluene was used whilst in Example 6 the alkaline treated dinitrotoluene feed was spiked with nitric acid to a concentration of 10,000 ppm (by weight) which approximated the acid concentration of the crude dinitrotoluene prior to water washing.

The results are given in Table 4.

TABLE 4

| | Example 5 WW DNT | Example 6 alkaline treated DNT with 10,000 ppm $HNO_3$ |
|---|---|---|
| Initial activity (mol $H_2$/mon. g cat. $cm^3$) | $3.67 \times 10^{-5}$ | $6.0 \times 10^{-5}$ |
| Final activity (mol $H_2$/mon. g cat. $cm^3$) | $3.67 \times 10^{-5}$ | $2.03 \times 10^{-5}$ |
| Tar generation (wt. %) | 1.7 | 30 ppm $HNO_3$ |
| Catalyst life gm DNT/gm cat. | 1800 | 220 |
| Nitrobody in reactor (ppm) | 5 | 5500 |

The procedure of Example 6 was repeated using different amounts of acid. It was found that above 6000 ppm $HNO_3$ the tar generation increased dramatically from 10 to 30 weight % product whereas at or below 6000 ppm the tar generation was typically 4 ppm. Similarly above 6000 ppm $HNO_3$ the nitrobodies in the reactor were typically about 5500 ppm whereas below 6000 ppm they were typically 5 ppm. Furthermore the catalyst life and activity decreased markedly as the concentration of $HNO_3$ increased above 6000 ppm.

## Claims

1. A method of manufacturing toluene diamine from crude dinitrotoluene containing acid together with *inter alia* nitrophenolic material characterized in that it comprises the steps of washing said crude dinitrotoluene with water until the concentration of acid in the crude dinitrotoluene is less than 6000 ppm (by weight) (based on $HNO_3$), and reacting the washed crude dinitrotoluene with hydrogen in a reactor in the presence of a catalyst without any intermediate step to reduce the concentration of nitrophenolic material present.

2. A method according to Claim 1, characterized in that the crude dinitrotoluene is washed with water until the concentration of acid in the crude dinitrotoluene is reduced to less than 2500 ppm (by weight) (based on $HNO_3$).

3. A method according to Claim 1 or 2, characterized in that said toluene diamine is manufactured continuously in said reactor.

4. A method according to Claim 3, characterized in that the concentration of dinitrotoluene in the contents of said reactor is maintained between 0.0001 and 25% (by weight).

5. A method according to Claim 4, characterized in that the concentration of dinitrotoluene in the contents of said reactor is maintained between 0.01 and 3% (by weight).

6. A method according to Claim 3, 4 or 5, characterized in that the concentration of catalyst in the contents of said reactor is maintained between 0.1 and 20% (by weight).

7. A method according to Claim 6, characterized in that the concentration of catalyst in the contents of said reactor is maintained between 0.1 and 3% (by weight).

8. A method according to any preceding Claim, characterized in that the catalyst is Raney nickel.

## Revendications

1. Procédé de préparation de la toluène diamine à partir de dinitrotoluène brut contenant de l'acide avec, entre autres, une matière nitrophènolique, caractérisé en ce qu'il comprend le lavage de ce dinitrotoluène brut avec de l'eau jusqu'à ce que la concentration de l'acide dans le dinitrotoluène brut soit inférieure à 6000 ppm (en poids) (sur la base de $HNO_3$), et la réaction du dinitrotoluène brut lavé avec de l'hydrogène dans un réacteur en présence d'un catalyseur sans aucune opération intermédiaire pour réduire la concentration de la matière nitrophénolique présente.

2. Procédé suivant la revendication 1, caractérisé en ce qu'on lave le dinitrotoluène brut à l'eau jusqu'à ce que la concentration de l'acide dans le dinitrotoluène brut soit réduite à moins de 2500 ppm (en poids) (sur la base de HNO₃).

3. Procédé suivant l'une quelconque des revendications 1 ou 2, caractérisé en ce que cette toluène diamine est préparée en continu dans ce réacteur.

4. Procédé suivant la revendication 3, caractérisé en ce que la concentration du dinitrotoluène dans le contenu de ce réacteur est maintenue entre 0.0001 et 25% (en poids).

5. Procédé suivant la revendication 4, caractérisé en ce que la concentration du dinitrotoluène dans le contenu de ce réacteur est maintenue entre 0,01 et 3% (en poids).

6. Procédé suivant l'une quelconque des revendications 3, 4 ou 5, caractérisé en ce que la concentration du catalyseur dans le contenu de ce réacteur est maintenue entre 0,1 et 20 % (en poids).

7. Procédé suivant la revendication 6, caractérisé en ce que la concentration du catalyseur dans le contenu de ce réacteur est maintenue entre 0,1 et 3% (en poids).

8. Procédé suivant l'une quelconque des revendications précédentes, catactérisé en ce que le catalyseur est du nickel de Raney.

### Patentansprüche

1. Ein Verfahren zur Herstellung von Toluoldiamin aus rohem Dinitrotoluol, das Säure zusammen mit unter anderem nitrophenolischem Material enthält, dadurch gekennzeichnet, daß es die Stufen des Waschens des genannten rohen Dinitrotoluols mit Wasser, bis die Konzentration der Säure in dem rohen Dinitrotoluol weniger als 6,000 ppm (in Gewichtsteilen) (bezogen auf HNO₃) beträgt, und Umsetzens des gewaschenen rohen Dinitrotoluols mit Wasserstoff in einem Reaktor in Gegenwart eines Katalysators umfaßt, ohne daß ein Zwischenschritt zur Verminderung der Konzentration des Vorliegenden nitrophenolischen Materials durchgeführt wird.

2. Ein Verfahren nach Anspruch 1, dadurch gekennzeichnet, daß das rohe Dinitrotoluol mit Wasser gewaschen wird, bis die Konzentration der Säure in dem rohen Dinitrotoluol auf weniger als 2,500 ppm (in Gewichtsteilen) (bezogen auf HNO₃) vermindert ist.

3. Ein Verfahren nach Anspruch 1 oder 2, dadruch gekennzeichnet, daß das genannte Toluoldiamin in dem genannten Reaktor kontinuierlich hergestellt wird.

4. Ein Verfahren nach Anspruch 3, dadurch gekennzeichnet, daß die Konzentration an Dinitrotoluol im Inhalt des genannten Reaktors zwischen 0,0001 und 25% (gewichts-%) gehalten wird.

5. Ein Verfahren nach Anspruch 4, dadurch gekennzeichnet, daß die Konzentration an Dinitrotoluol im Inhalt des genannten Reaktors zwischen 0,01 und 3% (gewichts-%) gehalten wird.

6. Ein Verfahren nach Anspruch 3, 4 oder 5, dadurch gekennzeichnet, daß die Konzentration des Katalysators im Inhalt des genannten Reaktors zwischen 0,1 und 20% (Gewichts-%) gehalten wird.

7. Ein Verfahren nach Anspruch 6, dadurch gekennzeichnet, daß die Konzentration des Katalysators im Inhalt des genannten Reaktors zwischen 0,1 und 3% (Gewichts-%) gehalten wird.

8. Ein Verfahren nach einem beliebigen vorangehenden Anspruch, dadurch gekennzeichnet, daß der Katalysator Raney-Nickel ist.